(19) Europäisches Patentamt
European Patent Office
Office européen des brevets

(11) **EP 3 235 428 B1**

(12) **EUROPEAN PATENT SPECIFICATION**

(45) Date of publication and mention
of the grant of the patent:
**29.07.2020 Bulletin 2020/31**

(51) Int Cl.:
*A61B 5/103* (2006.01)    *A61B 5/00* (2006.01)
*A43D 1/06* (2006.01)    *A43B 3/00* (2006.01)
*G01L 1/14* (2006.01)

(21) Application number: **16166283.8**

(22) Date of filing: **20.04.2016**

(54) **FLEXIBLE PRESSURE MAPPING DEVICE AND SYSTEM FOR MONITORING PRESSURE**

FLEXIBLE DRUCKABBILDUNGSVORRICHTUNG UND SYSTEM ZUR ÜBERWACHUNG VON DRUCK

DISPOSITIF DE MAPPAGE DE PRESSION FLEXIBLE ET SYSTÈME DE SURVEILLANCE DE PRESSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.10.2017 Bulletin 2017/43**

(73) Proprietor: **Feetme**
**75019 Paris (FR)**

(72) Inventors:
• **MATHIEU, Alexis**
**75011 Paris (FR)**
• **MOSTOVOV, Andrey**
**93300 Aubervilliers (FR)**
• **FOURNIER, Maximilien**
**75015 Paris (FR)**

(74) Representative: **Icosa**
**83 avenue Denfert-Rochereau**
**75014 Paris (FR)**

(56) References cited:
WO-A1-2016/009151    US-A- 4 745 930
US-A- 4 862 743    US-A- 5 323 650
US-A1- 2013 213 145    US-A1- 2014 174 205
US-B1- 6 195 921

## Description

FIELD OF THE INVENTION

The invention is generally related to the monitoring of pressure. More particularly, the invention relates to flexible pressure measurement devices.

One specific and non-limitative application of the invention is insoles for monitoring pressure and systems for monitoring pressure comprising such insoles.

BACKGROUND OF THE INVENTION

In medical fields or sport training, it is often desirable to know the distribution of static or dynamic pressure forces exerted by the body of a person.

One specific and non-limitative example of application in the medical field is related to mapping the pressure exerted by the body of diabetic patient with sensory neuropathy. Sensory neuropathy is a damage or disease affecting sensory nerves of a person which may cause numbness to touch.

Sensory neuropathy can have severe side effects. Indeed, since the afflicted person become unaware of the pressure in certain portion of its body it may cause dangerously high or prolonged pressure on its skin, for instance on sharp edges or rocks, that may damage the skin. One example is being unaware of a stone in a shoe and walking over said stone for a long period of time. A continuous monitoring of abnormal foot pressures may thus allow detecting the occurrence of such events and alert the wearer. Foot pressure monitoring can find many other applications as diagnostic for podiatry or orthopaedics.

In sports, a pressure mapping device worn by a sportsman and connected to a server can allow an athlete to quantify his or her moves and improve his or her performances.

To accurately monitor the pressure exerted by a portion of the body of the wearer, a pressure mapping device is usually made flexible in order to follow the movements of the wearer, for instance to follow the sole of the feet during walking.

For instance, WO 2009/070782 describes a force sensing system that can be used to sense pressure at a plurality of points of a user's foot and that comprise several transducers, processing means for processing the data acquired by the sensors and transmission means for transmitting the data to a remote server.

Documents US 2013/213145 and WO 2009/089406 also describe insoles including an array of pressure sensors together with signal processing means and a wireless transmitter able to relay the acquired signals to a remote.

However, these pressure mapping devices present several drawbacks.

Indeed, a flexible pressure mapping device is subjected to high mechanical stress over long period of times. Under regular use, such devices suffer strong stresses and structural constraints arising from the numerous bending cycles.

There are thus needs to improve the reliability and durability of flexible pressure mapping devices, while at the same time provide pressure mapping devices with a low profile and/or a low cost.

The instant invention has thus notably for object to improve this situation.

SUMMARY OF THE INVENTION

To this aim, a first object of the invention is a flexible pressure mapping device comprising:

- a flexible upper sheet and a flexible lower sheet facing one another;
- a flexible middle sheet extending between the upper sheet and the lower sheet;
- a plurality of force sensors comprising at least one upper electrode on the upper sheet and at least one lower electrode on the lower sheet, said at least one upper electrode facing said at least one lower electrode and being separated from said lower electrode at least by the middle sheet;
- at least one upper conductive lead on the upper sheet, electrically connected to the at least one upper electrode, and at least one lower conductive lead on the lower sheet, electrically connected to the at least one lower electrode;
- control and transmit electronics electrically connected to the at least one upper conductive lead and to the at least one lower conductive lead, wherein the upper sheet comprises a plurality of upper openings, and the lower sheet at least partially overlaps at least one of said upper openings, the lower sheet comprising a plurality of lower openings, the device comprising at least one double-opening region, in which at least one upper opening of the plurality of upper openings or one lower opening of the plurality of lower openings overlaps at least partially one middle opening of the middle sheet, no opening being provided on one of the lower sheet and upper sheet in said at least one double-opening region, the device further comprising an upper overcoat sheet and a lower overcoat sheet both extending in a horizontal plane (X, Y) and respectively arranged on top of the upper sheet and below the lower sheet.

With these features, it is possible to increase the flexibility and thus the durability of the flexible pressure mapping device by designing regions of the device with increased mechanical resistance to bending while maintaining the mechanical integrity of the device.

The invention is defined by appended claims 1-12.

In some exemplary embodiments, one might also use one or more of the following features:

- at least one upper opening at least partially overlaps with said double-opening region, thereby defining a triple-opening region;
- the device further comprises a plurality of upper electrodes on the upper sheet connected by a network of upper conductive leads of the upper sheet in a matrix arrangement such that each upper conductive lead connects a row of upper electrodes substantially aligned along a transverse direction of the horizontal plane,

  and the upper openings extend substantially along the transverse direction;
- the device further comprises a plurality of lower electrodes on the lower sheet connected by a network of lower conductive leads of the lower sheet in a matrix arrangement such that each lower conductive lead connects a column of lower electrodes substantially aligned along a longitudinal direction of the horizontal plane,

  and the lower openings extend substantially along the transverse direction;
- at least one of the upper openings extends along a majority of a transversal extend of the device;
- at least one of the upper openings is an opening with a closed contour;
- the device further comprises at least one ground plane extending in the horizontal plane over a majority of the device;
- the device further comprises an upper ground plane on the upper sheet and a lower ground plane on the lower sheet arranged such that the upper electrodes, the middle sheet and the lower electrodes are arranged between the lower ground plane and the upper ground plane;
- the flexible middle sheet is insulating and the force sensors are capacitive force sensors;
- the device further comprises an upper overcoat sheet extending in the horizontal plane and arranged on top of the upper sheet, and at least a portion of said upper overcoat sheet is able to slide in the horizontal plane with regard to the upper sheet;
- the flexible pressure mapping device is an insole for insertion into an article of footwear or is integrated in an article of footwear;
- the device further comprises an upper contacting layer on top of the upper overcoat sheet of the device, said upper contacting layer being elastic and adapted to be engaged by a foot of a wearer.

Another object of the invention is a system for monitoring pressure comprising:

  a flexible pressure mapping device as detailed above, and
  a remote server able to communicate with the control and transmit electronics of the device to receive pressure related data from the device.

BRIEF DESCRIPTION OF THE DRAWINGS

Other characteristics and advantages of the invention will readily appear from the following description of several of its exemplary embodiments, provided as non-limitative examples, and of the accompanying drawings. The invention being defined by appended claims 1-12.

On the drawings:

- Figure 1 is a schematic illustrating a system according to an exemplary embodiment of the invention comprising a pressure mapping device according to an embodiment of the invention and a remote server, where the upper contacting layer and the upper ground plane have been hidden in order to show the force sensors of the device.
- Figure 2 is an exploded view of the pressure mapping device of figure 1 illustrating an overcoat adhesive, an upper overcoat sheet, an upper adhesive, an upper sheet, a middle sheet, a lower sheet, a lower adhesive, a lower overcoat sheet and an overcoat adhesive.
- Figure 3 is a detailed transverse section of the pressure mapping device of figure 1.
- Figures 4A, 4B, 4C, 4D and 4E are detailed top view of, respectively, an upper sheet, a lower sheet, a middle sheet, an upper overcoat sheet and an upper adhesive of the pressure mapping device of figure 1.

On the different figures, the same reference signs designate like or similar elements.

DETAILED DESCRIPTION

The invention is defined by appended claims 1-12. Exemplary embodiments are shown in the drawings and herein described in details with the understanding that the present disclosure is to be considered as an exemplification of the principles of the invention.

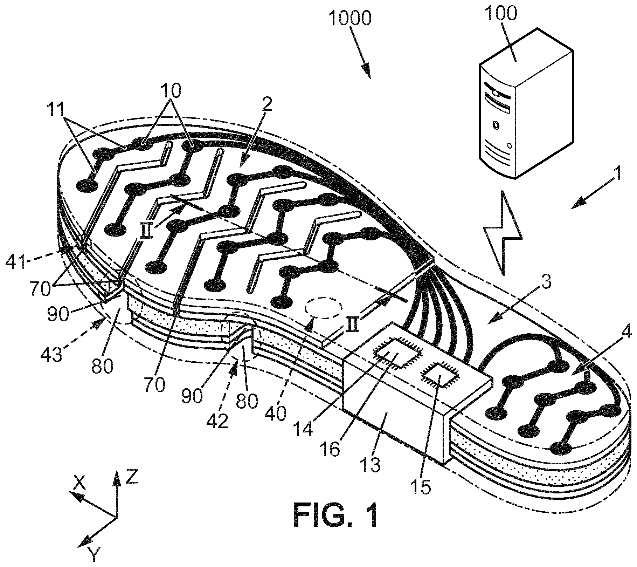

Referring to figure 1, there is shown a flexible pressure mapping device 1 according to an examplary embodiment.

One non-limitative example of application of the flexible pressure mapping device 1 is an insole intended to be inserted into an article of footwear or included in the structure of the footwear. The article of footwear may for instance be a shoe and can take many forms such that a street, athletic or orthopaedic shoe.

Other applications can also be considered, for instance for mapping pressure of the hand, finger, toes, legs, waist, hips, butt, back, etc.

As illustrated on figure 1, the pressure mapping device 1 is substantially planar and extends perpendicularly to a thickness direction Z.

The device 1 may thus extend between an upper surface 1a and a lower surface 1b, facing each other, both surfaces being perpendicular to the thickness direction Z.

Unless stated otherwise, the terms "top", "bottom", "up", "down", "upper", "lower", "above", "below" and the like refers to spatial position and orientation with regards to the thickness direction Z. It should be noted that the orientation of the thickness direction Z disclosed in the present disclosure is not limitative. In particular, the top and bottom of the device should be understood as conventional orientations and not as absolute references to a vertical axis of the environment.

By "substantially planar", it is understood that the device has the general shape of a plane sheet at a local level and, at a local level, extends generally along a plane, having large dimensions along a longitudinal direction X and a transverse direction Y (transverse direction Y being perpendicular to the longitudinal direction X), and a smaller dimension along a thickness direction Z that is perpendicular to the longitudinal and the transverse directions X, Y. At a local level, the extension of the device along the thickness direction Z may thus be at least ten times smaller than the extension of the device 1 in the longitudinal and the transverse directions X, Y.

By a "local level", it is understood a small region of the device, as opposed to a "global level" which corresponds to the device considered in its entirety.

Since the device is flexible it may exhibit, at a global level, a shape that departs from a plane. The device may thus present structures, bumps and curves and departs from a plane. The device may for instance be wrapped around an external shape and conform to said shape, for instance a rounded shape.

In one specific and non-limitative example, the device may for instance be inserted in an orthopaedic shoes and conform the insole of said orthopaedic shoes.

The longitudinal and the transverse directions X, Y form a plane denoted here as a "horizontal plane" X, Y.

More particularly, the device 1 may extend mainly along the longitudinal direction X, secondly along the transverse direction Y. For instance, the device has a length measured along the longitudinal direction X and a width measured along transverse direction Y, the length of the device being greater than the width of the device.

As a non-limitative example, the extension of the device 1 along the transverse direction Y, called the "width", may be smaller than half of the extension of the device 1 along the longitudinal direction X, called the "length".

In the present description the extension of the device along the thickness direction Z is called "thickness". By "thickness", it is thus understood a maximal distance separating the upper surface 1a and the lower surface 1b of the device 1, said distance being measured along the thickness direction Z.

In a non-limitative example, the extension of the device 1 along the thickness direction Z may be smaller than ten times the extension of the device 1 along the longitudinal direction X. The extension of the device 1 along the thickness direction Z may be smaller than several times the extension of the device 1 along the transverse direction Y, for instance smaller than five times the extension of the device 1 along the transverse direction Y.

The device 1 may for instance present a thickness of less than two centimetres, preferably less than 1,5 centimetres, for instance about 1 centimetre.

As illustrated on figure 1, in one specific and non-limitative example, the device 1 may comprise a front portion 2 arranged to be engaged by a forefoot of a foot, a mid portion 3 arranged to be engaged by a midfoot of the foot, and a rear portion 4 arranged to be engaged by a hindfoot of the foot.

The front portion 2, the mid portion 3 and the rear portion 4 are connected together to form a single element that may be more or less flexible.

The front portion 2 may in particular extend on a larger dimension than the mid portion 3 along the transverse direction Y, i.e. the width of the front portion 2 may be greater than the width of the mid portion 3.

The device 1 comprises a laminated sheet-like element with several stacked layers. This laminated sheet-like element may be embedded in other materials, for instance by injecting polyurethane around the stacked layers or by sticking additional layer on top or below the laminated sheet-like element.

The configuration of layers of the device 1 can in particular vary along its longitudinal and transverse extension as it will now be further detailed.

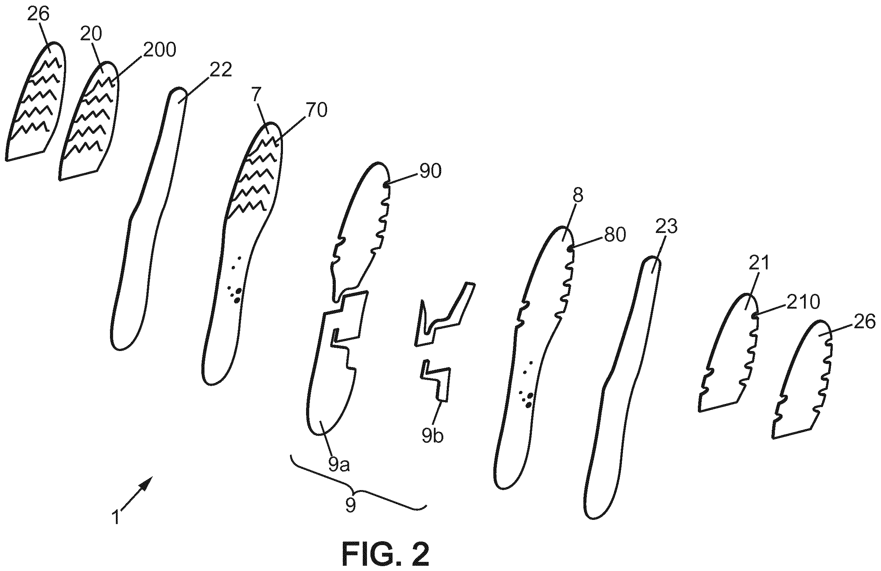

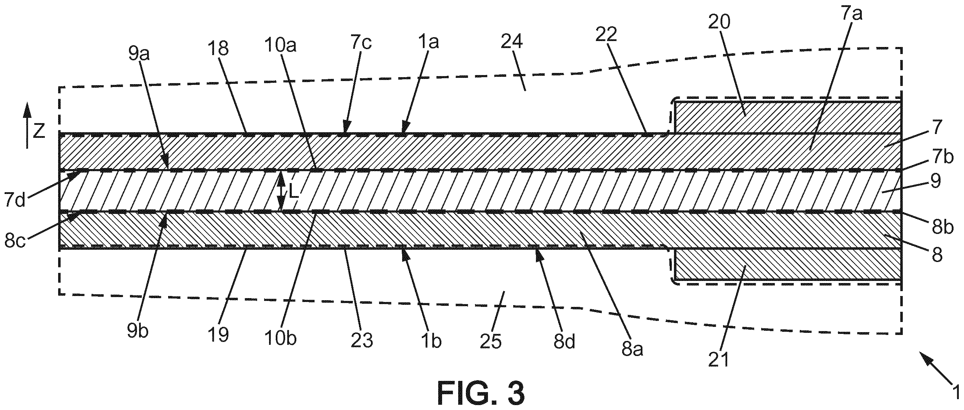

Referring now to figure 2 and 3, the device 1 comprises a flexible upper sheet 7 and a flexible lower sheet 8. The upper sheet 7 and the lower sheet 8 both extend generally along the longitudinal and transverse directions X, Y. The upper sheet 7 and the lower sheet 8 thus face each other in the thickness direction Z.

The device 1 further comprises a middle sheet 9. The middle sheet 9 arranged between the upper sheet 7 and the lower sheet 8.

The middle sheet 9 is advantageously a dielectric sheet, in particular a flexible insulating sheet as it will be detailed after.

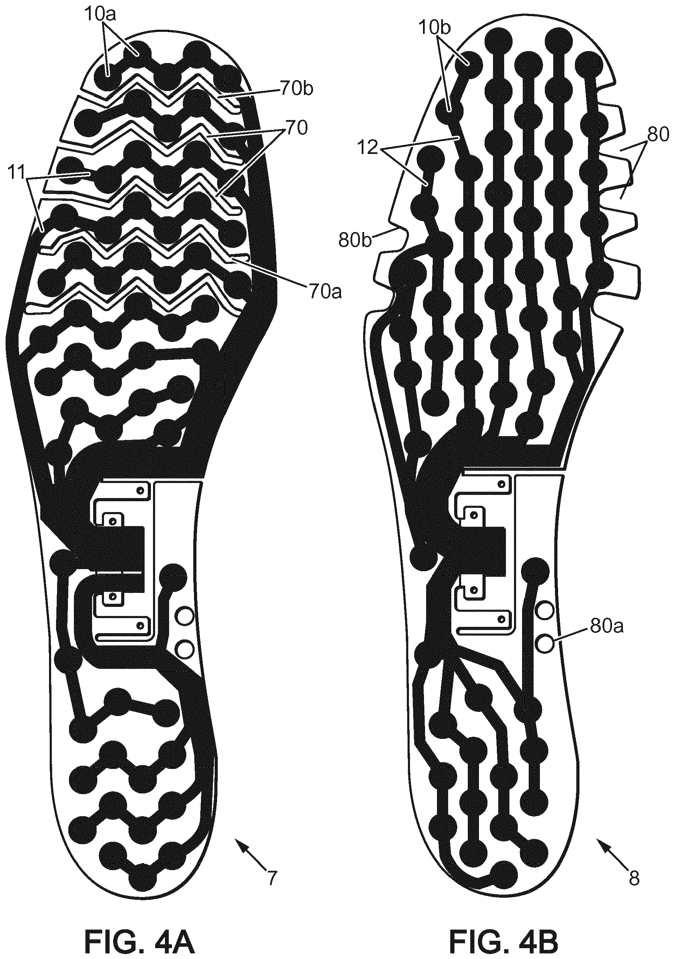

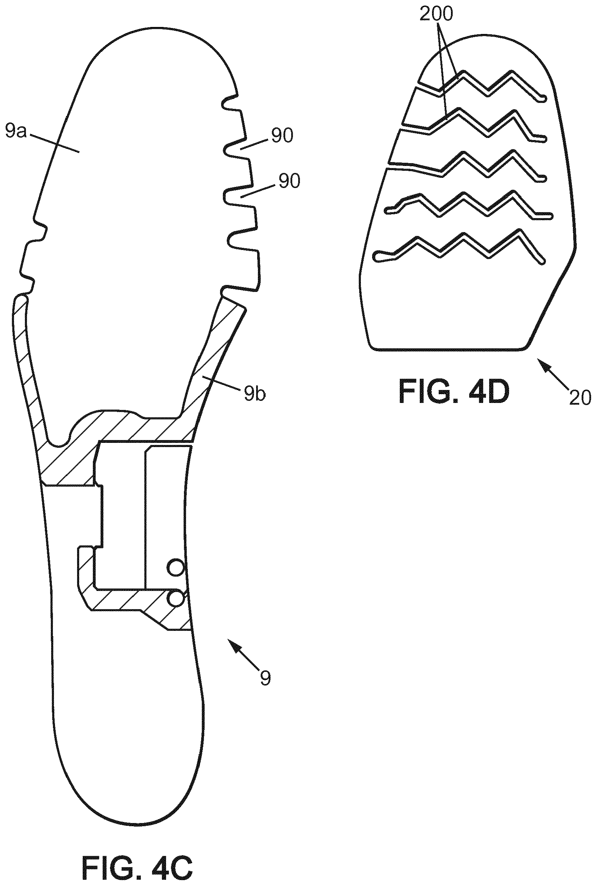

As illustrated on figures 1-4E, a network of force sensors 10 is provided on the device 1. The force sensors 10 are for instance located in the front portion 2, the mid portion 3 and the rear portion 4 of the device 1.

The plurality of force sensors 10 comprises at least one upper electrode 10a on the upper sheet 7 and at least one lower electrode 10b on the lower sheet 8.

In particular, the plurality of force sensors 10 may comprise a plurality of upper electrodes 10a on the upper sheet 7 and a plurality of lower electrodes 10b on the lower sheet 8.

Referring more particularly to figure 3, each force sensor 10 comprises an upper electrode 10a on the upper sheet 7 and a lower electrode 10b on the lower sheet 8. The upper electrode 10a and the lower electrode 10b extend perpendicularly to the thickness direction Z and faces each other in the thickness direction Z.

For example, the upper electrode 10a and the lower electrode 10b may be squares with about 5 mm sides, or may be disks with few millimetres diameters.

The upper electrode 10a and the lower electrode 10b are separated from each other at least by the middle sheet 9.

The force sensors 10 can be compression force and/or shear force sensors.

In one particular example, the force sensors 10 are capacitive force sensors.

In this embodiment, the middle sheet 9 is advantageously an insulating dielectric sheet.

Alternatively, the force sensors 10 may also be force sensing resistor. In this example, the middle sheet 9 is advantageously a conducting or semi-conducting sheet. The middle sheet may for instance be a conducting adhesive sheet and/or a conductive polymer which changes resistance in a predictable manner following application of force to its surface. Such a conductive polymer may for instance comprise electrically conducting and nonconducting particles suspended in matrix.

A network of upper conductive leads 11 is provided on the upper sheet 7. The upper conductive leads 11 are electrically connected to the upper electrodes 10a of the force sensors 10. Similarly, a network of lower conductive leads 12 is provided on the lower sheet 8. The lower conductive leads 12 are electrically connected to the lower electrodes 10b of the force sensors 10.

The network of conductive leads and sensors can be covered by an additional dielectric layer, protecting the leads and sensors electrically and mechanically.

In one example, the networks of upper conductive leads 11 and lower conductive leads 12 may be spatially multiplexed, i.e. arranged to be able to address each capacitive force sensor 10 separately by a combination of an upper conductive lead 11 and a lower conductive lead 12, for instance by addressing rows and columns in a matrix arrangement of the force sensors 10.

Alternatively, one of the upper conductive leads 11 and the lower conductive leads 12 may be arranged to connect separately each capacitive force sensor 10.

To this aim, the upper sheet 7 and the lower sheet 8 each comprise at least one conductive layer 7b, 8b. The upper sheet 7 and the lower sheet 8 also comprise each at least one insulating layer 7a, 8a.

The upper sheet 7 and the lower sheet 8 may be each provided with at least one conductive layer 7b, 8b. In the example of figure 3, the upper sheet 7 and the lower sheet 8 are each provided with a single insulating layer 7a, 8a.

Insulating layers 7a, 8a are made of insulating flexible polymer and may for instance be each made of a material selected in the list comprising polyester, polyimide, polyethylene napthalate, Polyetherimide, fluropolymers, mixed cells polyurethane foam, polyurethane, thermoplastic polyurethane, thermoplastic polymer such as "Hytrel", polyamide and copolymers of the formers. Insulating layers 7a, 8a can also be made of textiles.

For instance, the insulating layers 7a, 8a may be made of a polyethylene terephthalate (also called "PET") film. The insulating layers 7a, 8a are for instance PET sheet with a thickness between 50 and 100 microns.

The conductive layer 7b, 8b are electrically conductive and can be deposited on the insulating layers 7a, 8a or printed with an electrically conductive ink. The conductive layer 7b, 8b may be made or metal or conductive polymer for instance.

The conductive layer 7b of the upper sheet 7 comprises the upper electrodes 10a of the force sensors 10 and the network of upper conductive leads 11. Similarly, the conductive layer 8b of the lower sheet 8 comprises the lower electrodes 10b of the force sensors 10 and the network of lower conductive leads 12.

In one preferred example, the upper sheet 7 and the lower sheet 8 are insulating except for the conductive leads 11, 12 and the electrodes 10a, 10b of force sensors 10.

In the example illustrated on figure 3, the insulating middle sheet 9 extends between an upper face 9a and a lower face 9b. The upper sheet 7, the middle sheet 9 and the lower sheet 8 are arranged in a coplanar staking arrangement as follows. The upper sheet 7 is laminated on the upper face 9a of the middle sheet 9 so that the conductive layer 7b is in surface contact with the upper face 9a of the middle sheet 9. On the other side of the middle sheet 9, the lower sheet 8 is laminated on the lower face 9b of the middle sheet 9 so that the conductive layer 8b is in surface contact with the lower face 9b of the middle sheet 9.

In other words, the upper sheet 7 and the lower sheet 8 each have respective upper faces 7c, 8c and respective lower faces 7d, 8d, facing the upper faces in the thickness direction Z. The conductive layer 7b of the upper sheet 7 is located on its lower faces 7d and the conductive layer 8b of the lower sheet 8 is located on its upper faces 8c.

The lower face 7d of the upper sheet 7 and the upper face 8c of the lower sheet 8 are thus in contact with the middle sheet 9 of the device 1. The upper electrodes 10a of the force sensors 10 and the upper network of conductive lead 11 are then located on the lower face 7d of the upper sheet 7. The lower electrodes 10b of the force sensors 10 and the lower network of conductive lead 12 are located on the upper face 8c of the lower sheet 8.

Other examplary embodiments can be envisaged, wherein the conductive layers 7b, 8b and insulating layers 7a, 8a of the upper sheet 7 and the lower sheet 8 are arranged differently and, in particular are switched in the thickness direction Z.

Moreover, as illustrated on figure 3, the device may also comprise at least one ground plane 18, 19. Said ground plane 18, 19 may extend in the horizontal plane X, Y over a majority of the area of the device 1.

The ground plane 18, 19 may not be a full plane but can also be, or comprise, a network of ground lines such as a grid, for instance a network of lines separated by a distance of about half a distance separating a row or a column of a matrix arrangement of the force sensors 10 as detailed above.

The ground plane may in particular be a floating ground plane, in that said ground plane may be free from electrical connection, in particular free from electrical connection with the control and transmit electronics 14 of the device.

The ground plane may be conductive. The ground plane can be deposited on the insulating layers 7a, 8a or printed with an electrically conductive ink, thereby forming a conductive layer 7b, 8b of the upper sheet 7 and/or lower sheet 8. The conductive layer 7b, 8b may be made or metal, conductive polymer or carbon for instance.

In particular, the device 1 may comprise an upper ground plane 18 and a lower ground plane sheet 19 both extending in the horizontal plane X, Y over a majority of the device area.

The upper ground plane sheet 18 and a lower ground plane sheet 19 may be arranged such that the upper electrodes, the middle sheet 9 and the lower electrodes are arranged between the upper ground plane 18 and the lower ground plane 19.

In one example, illustrated on figure 3, the upper electrodes 10a are located on the lower face 7d of the upper sheet 7 and the upper ground plane 18 is then located on the upper face 7c of the upper sheet 7.

In this example, the lower electrodes 10b may then be located on the upper face 8c of the lower sheet 8 and the lower ground plane 19 may be located on the lower face 8d of the lower sheet 8.

In the absence of tensile or compressive loads or lateral shear, the value of the capacitance C of a capacitive force sensor 10 can be determined as a function of the thickness L of the middle sheet 9 at the location of the capacitive force sensor 10, the surface S of the upper electrode 10a and the lower electrode 10b and the relative permittivity $\varepsilon_r$ of the material between the electrodes, in particular the middle sheet 9, in particular by the following equation ($\varepsilon_0$ being vacuum permittivity):

$$C = \frac{\varepsilon_r \varepsilon_0 S}{L}$$

Under the effect of a compressive or tensile load, the thickness L of the middle sheet 9 at the location of the capacitive force sensor 10 is changed and the capacitance C of the sensor 10 thus varies.

The thickness of the middle sheet 9 along the thickness direction Z may be in the range 0.2 mm to 1 mm.

The middle sheet 9 is advantageously made of a dielectric material which is resiliently deformable under tensile loads, compressive loads and lateral shearing. The mechanical resilience of a material elastomer can be defined as a ratio, often expressed as a percentage, of the energy returned after deformation divided by the energy used to deform the elastomer, under cyclic loading. The hysteresis rate corresponds to the dissipated energy and is thus the complement of the mechanical resilience. A high resilience can be associated to a low hysteresis.

The material of the middle sheet 9 can be selected depending on the application. The middle sheet 9 may for instance be made in a material selected in a list comprising cork, micro-architectured cork, elastomer, rubber, urethane, silicone, butyl rubber, polymer, neoprene, closed cells or open cells polyurethane foam, polyisoprene foam, and urethane foam.

In particular, in an example where the force sensors 10 are capacitive force sensors, the middle sheet 9 may be made of an insulating material. The middle sheet may thus be made of a material that is neither conducting nor semi-conducting.

In a preferred exemplary embodiment, the middle sheet 9 remains within the elastic deformation range for applied pressures between 0 and 10 kg/cm (corresponding to the average of the plantar pressure) and the crushing of the material is preferably limited to between 10% and 50% of the thickness, preferably less than 50% when the applied pressure is 15 kg/cm.

This way, when the output capacity of the electrodes 10a, 10b is measured before and after the application of a compression load, it is possible to compute a variation in thickness L of the middle sheet 9 at the location of the capacitive force sensor 10.

In particular, it is then possible to compute a normal pressure applied at the location of the capacitive force sensor 10, for instance by using for instance Hook's law which indicates that the normal strain σ applied to the surface of the middle sheet 9 is equal to the product of the percentage of thickness variation ΔL/L by the Young's modulus E of the material of the middle sheet 9:

$$\sigma = E \frac{\Delta L}{L}$$

The middle sheet 9 can for instance present a Young's modulus between 1 MPa and 5 MPa and a Poisson's ratio between 0 and 0.5 and preferably less than 0.1. The middle sheet 9 may have a relative permittivity between 2 and 20.

In one example, the middle sheet 9 is made of at least two different materials, a first middle material 9a and a second middle material 9b.

The first middle material 9a may be used at least provided inside the force sensor 10, i.e. between the upper and lower electrodes 10a, 10b of the force sensors 10.

The second middle material 9b may be more rigid than the first middle material 9a and/or more resistant to crushing.

The second middle material 9b may be provided in area of the device where there are high densities of conductive leads 11, 12. The crushing of the second material 9b may be preferably limited to between 5% and 10% of the thickness, preferably less than 10% when the applied pressure is 15 kg/cm.

The second middle material 9b may for instance be present near the control and transmit electronics 14of the device 1 that is described further below.

As illustrated on figure 1 and 4A, the upper sheet 7 comprises a plurality of upper openings 70.

The upper openings 70 are arranged such that the lower sheet 8 overlaps at least a portion of the plurality of said upper openings 70.

As illustrated on figure 1 and 4B, the lower sheet 8 may also comprises a plurality of lower openings 80.

The upper sheet 7 may also overlap at least a portion of said plurality of said lower openings 80.

In one example, said lower sheet 8, resp. upper sheet 7, may overlap completely at least one upper opening 70 from the plurality of upper openings 70, resp. at least one lower opening 80 from the plurality of lower openings 80.

In another example, said lower sheet 8, resp. upper sheet 7, may overlap only partially at least one upper opening 70 from the plurality of upper openings 70, resp. at least one lower opening 80 from the plurality of lower openings 80.

By "a sheet overlaps at least a portion of a plurality of openings", it is meant that at least a portion of said sheet is facing at least a portion of an upper opening.

Said overlap between the lower sheet 8, resp. upper sheet 7, and an upper opening 70, resp. a lower opening 80, is referred to as a single-opening region 41 of the device.

In such a single-opening region 41, only one of the upper sheet, middle sheet and lower sheet has an opening.

The device according to the invention may also comprise at least one double-opening region 42, which can be defined as follows.

The middle sheet 9 may comprise at least one middle opening 90, in particular a plurality of middle openings 90.

The middle openings 90 may be located outside of the force sensors 10, in order to avoid disturbing the force measurement.

A double-opening region 42 is thus illustrated on figure 1. In a double-opening region 42, at least one upper or lower opening 70, 80 of the plurality of upper openings 70 or of the plurality of lower openings 80 overlaps at least partially a middle opening 90 of the middle sheet 9.

In such a double-opening region, no opening is provided on one the lower sheet and upper sheet which sheet thus maintains the mechanical integrity of the device.

By "an upper opening overlaps at least partially a middle opening" or "a lower opening overlaps at least partially a middle opening", it is meant that at least a portion of said upper opening or said lower opening faces at least a portion of said middle opening.

In such a double-opening region 42, both the upper sheet and middle sheet are absent, or both the lower sheet and the middle sheet are absent.

In a (non-illustrated) variant, a double-opening region 42 may also be defined as a region in which at least one upper opening of the plurality of upper openings overlaps at least partially a lower opening of the lower sheet.

By "an upper opening overlaps at least partially a lower opening", it is meant that at least a portion of said upper opening faces at least a portion of said lower opening.

In such a double-opening region 42, both the upper sheet and lower sheet are absent. In such a double-opening region, no opening is provided on the middle sheet, which thus maintains the mechanical integrity of the device.

As illustrated on figure 1, the device may further comprise at least one triple overlap region 43.

In a triple overlap region 43, an upper opening 70, a middle opening 90 and a lower opening 80 at least partially overlap.

In such a triple-opening region 43, the upper sheet 7, the middle sheet 9 and the lower sheet 8 are all absent.

At least one full-sheets region 40 may also be defined, being a region 40 with no opening on any of the upper sheet, the middle sheet and the lower sheet. In a full-sheets region 40, the upper sheet 7, the middle sheet 9 and the lower sheet 8 are thus all presents.

Full-sheets regions 40, single-opening regions 41, double-opening regions 42 and triple-opening regions 43 are regions of the device with increasing mechanical resistance to bending and decreasing mechanical resistance to elongation in the horizontal plane.

These regions 40, 41, 42, 43 may thus be arranged along the device in order to increase the flexibility and thus the durability of the flexible pressure mapping device 1.

One example of arrangement of said full-sheets regions 40, single-opening regions 41, double-opening regions 42 and triple-opening regions 43 will now be described in details.

In the example of figure 4A, the upper electrodes 10a on the upper sheet 7 are connected by the network of upper conductive leads 11 in a matrix arrangement such that each upper conductive lead 11 connects a row of upper electrodes 10a substantially aligned along a transverse direction Y of the horizontal plane X, Y.

Alternatively, only a sub-set of the plurality of upper electrodes 10a may be organized in a row arrangement such a row of upper electrodes 10a of said sub-set are substantially aligned along a transverse direction Y of the horizontal plane X, Y and connected by an upper conductive leads 11.

In this embodiment, the upper openings 70 may then extend substantially on the upper sheet 7 along the transverse direction Y.

By "extend substantially along the transverse direction", it is understood that a majority of the upper openings 70 present a main extension axis more or less aligned with the transverse direction Y. As illustrated on figure 4A, the upper openings need not to be straight lines and may depart locally from the transverse direction Y.

At least one of the upper openings 70 may in particular extend along a majority of a transversal extent of the device 1.

In one example illustrated on figure 4A, an upper opening 70 can for instance extends with a zigzag shape between two rows of upper electrodes 10a.

The lower electrodes 10b on the lower sheet 8 may also be connected by the network of lower conductive leads 12 in a matrix arrangement. On the lower sheet 8, each lower conductive lead 12 may for instance connect a column of lower electrodes 10b substantially aligned along a longitudinal direction X of the horizontal plane X, Y.

As explained above, in a variant, only a sub-set of the plurality of lower electrodes 10b may be organized in a column arrangement such a column of lower electrodes 10b of said sub-set are substantially aligned along a longitudinal direction X of the horizontal plane X, Y and connected by a lower conductive lead 12.

In a variant, the upper electrodes 10a may be arranged in column, and the lower electrode may be arranged in rows.

In yet another variant, a first sub-set of the upper electrodes 10a may be arranged in column and a second sub-set of the upper electrodes 10a may be arranged in rows.

In this example also, the upper openings 70 may extend substantially on the lower sheet 8 along the transverse direction Y.

In this embodiment, both the upper openings 70 and the lower openings 80 extend substantially along the transverse direction Y. This example is particularly adapted to the use of the pressure mapping device as, or in, an insole since the bending of an insole mainly happens along the transverse direction Y as a result of folding the toes.

In other examples, the upper openings 70 and/or the lower openings 80 may also extend substantially along the longitudinal direction X.

At least one of the upper openings 70 may be an opening with a closed contour 70a.

As illustrated on figure 4A, the plurality of upper openings 70 may also comprises at least one upper opening 70 with an open contour 70b.

In the example of figures 4B, the plurality of lower openings 80 also comprises at least one lower opening 80 with an open contour 80b.

The plurality of lower openings 80 also comprises lower opening 80 with closed contours 80a.

By "an opening with a closed contour" it is understood that said opening forms a hole in the sheet.

By "an opening with an open contour" it is understood that said opening forms a notch in the sheet.

Openings with open contours make the device more resistant to bending while openings with closed contours are more resistant to elongation in the horizontal plane.

These openings may thus be arranged accordingly along the device in order to increase the durability of the flexible pressure mapping device.

In particular, the upper openings 70 and the lower openings 80 may present contours 70a, 70b, 80a, 80b with rounded corners. This way, the durability of the device can be further increased since the breaking lines on the device tend to extend from sharp corners in the contours of the sheets.

Moreover, the device 1 may also comprise at least one overcoat sheet 20, 21.

The overcoat sheet 20, 21 may extend in the horizontal plane X, Y and be arranged outside the staking arrangement of the upper sheet 7, the middle sheet 9 and the lower sheet 8.

The overcoat sheet 20, 21 may extend only on a portion of the longitudinal extent of the device. For instance, the overcoat sheet 20, 21 may extend only on half of the longitudinal extent of the device or extend on less than half of the longitudinal extent of the device.

The overcoat sheet 20, 21 may thus only be provided in the front portion 2 of the device 1, as illustrated on figures 1, 2, 3 and 4D.

In particular, the device 1 may comprise an upper overcoat sheet 20 and a lower overcoat sheet 21 both extending in the horizontal plane X, Y.

The upper overcoat sheet 20 may extend on top of the upper sheet 7 and the lower overcoat sheet 21 may extend below the lower sheet 8.

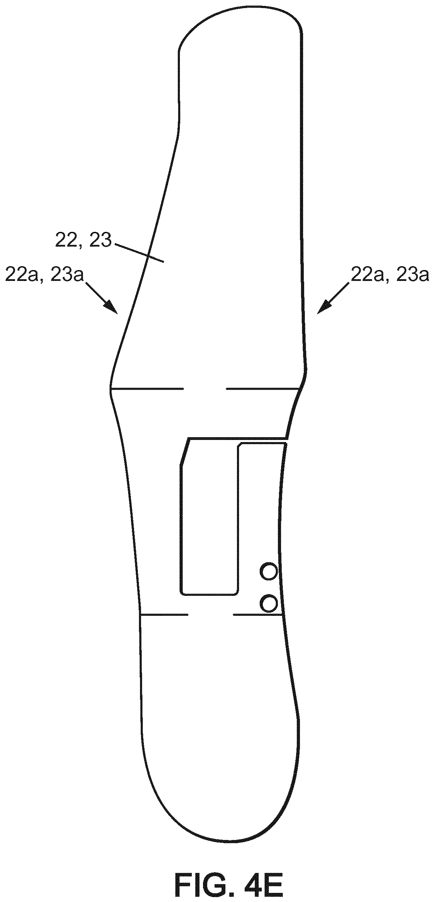

The upper overcoat sheet 20, resp. the lower overcoat sheet 21, may be attached to the upper sheet 7, resp. to the lower sheet 8, by respective layers of upper adhesive 22 and lower adhesive 23, as illustrated on figure 3 and 4E.

The upper overcoat sheet 20 may be provided with a plurality of upper overcoat openings 200. The plurality of upper overcoat openings 200 may be similar to the upper openings 70, at illustrated on figure 4D.

The lower overcoat sheet 21 may be provided with a plurality of lower overcoat openings 210. The plurality of lower overcoat openings 210 may be similar to the lower openings 80.

The respective layers of upper adhesive 22 and lower adhesive 23 may not covert the whole area of the device and may thus leave respective upper and lower adhesive-free regions 22a, 23a.

In the adhesive-free regions 22a, the upper sheet 7 and the upper overcoat sheet 20 are thus able to slide in the horizontal plane (X, Y) with regard to one another.

In the adhesive-free regions 23a, the lower sheet 8 and the lower overcoat sheet 21 are thus able to slide in the horizontal plane (X, Y) with regard to one another.

This way it is possible to relieve even better the tensile stress exerted on the flexible device when said device bends.

As illustrated on figure 3, the device may in particular be provided with an upper contacting layer 24. The upper contacting layer 24 may be arranged on top of the upper overcoat sheet 20 of the device 1. The upper contacting layer 24 may be in contact with a wearer and transmitting forces applied by said wearer.

The upper contacting layer 24 may thus be secured to the upper overcoat sheet 20, for instance by an adhesive so that the upper overcoat sheet 20 move together with the upper contacting layer 24.

The upper contacting layer 24 is elastic and is adapted to be engaged by a foot of a wearer.

Similarly, the device may further be provided with a lower contacting layer 25. The lower contacting layer 25 may be arranged below the lower overcoat sheet 22 of the device 1. The lower contacting layer 25 may be secured to the lower overcoat sheet 21, similarly to what has been described for the upper contacting layer 24 and the upper overcoat sheet 20.

The lower contacting layer 25 may be elastic. The lower contacting layer 25 may also be adapted to be engaged by a foot of a wearer so that identical pressure mapping device may be used as insoles for a left foot and a right foot of a wearer.

The upper contacting layer 24 and the lower contacting layer 25 may be respectively attached to the upper overcoat sheet 20 and to the lower overcoat sheet 21 by mean of an overcoat adhesive 26.

The device 1 also comprises control and transmit electronics 14.

The control and transmit electronics 14 include for instance a control electronic 15 and a wireless transceiver 16. The control electronic 15 comprises for instance at least one chip electrically connected to the force sensors 10. The wireless transceiver 16 is able to communicate with a remote server 100 as it will be detailed further below. In particular, the wireless transceiver 16 is connected to the control electronic 15 and is able to receive data from the control electronic 15 and transmit said data to the remote server 100 by wireless means (such as Wi-Fi, Bluetooth, sigfox ...) .

The device 1 may be further provided with a power supply module, for instance a battery and/or an energy harvesting module able to power the control and transmit electronics 14. The battery can advantageously be a flexible battery.

A chip support member 13 may be provided to strengthen the device 1 at the location of the control and transmit electronics 14 and allow the mounting of the control and transmit electronics 14 on the device 1.

The device 1 may also comprise a casing, for example a rigid plastic casing or a coating of polymer. The casing may comprise one or several openings, for instance an opening next to the wireless transceiver 16 of the control and transmit electronics 14. This way the quality of wireless data transmit may be improved.

With reference to figure 1, another object of the present invention is a system 1000 for monitoring pressure.

The system 1000 comprises a flexible pressure mapping device 1 as described above.

System 1000 also comprises a remote server 100. The remote server 100 is able to receive pressure related data from the device 1 by communicating with the wireless transceiver 16 of the device 1.

The remote server 100 may for instance be a computer, a smartphone or the like, with a wireless communication module.

The remote server 100 may be able to communicate over an extended network such as the internet in order to send the pressure related data and/or receive additional data from the network for post-processing.

By "pressure related data", it is meant data that are computed by the control electronic 15 on the basis of the measurements of the force sensors 10. The invention is defined by appended claims 1-12.

## Claims

1. A flexible pressure mapping device (1) comprising:

   - a flexible upper sheet (7) and a flexible lower sheet (8) facing one another;
   - a flexible middle sheet (9) extending between the upper sheet (7) and the lower sheet (8);
   - a plurality of force sensors (10) comprising at least one upper electrode (10a) on the upper sheet (7) and at least one lower electrode (10b) on the lower sheet (8), said at least one upper electrode (10a) facing said at least one lower electrode (10b) and being separated from said lower electrode (10b) at least by the middle sheet (9);
   - at least one upper conductive lead (11) on the upper sheet (7), electrically connected to the at least one upper electrode (10a), and at least one lower conductive lead (12) on the lower sheet (8), electrically connected to the at least one lower electrode (10b);
   - control and transmit electronics (14) electrically connected to the at least one upper conductive lead (11) and to the at least one lower conductive lead (12) ,

   wherein the upper sheet (7) comprises a plurality of upper openings (70),
   and the lower sheet (8) at least partially overlaps at least one of said upper openings (70),

the lower sheet (8) comprising a plurality of lower openings (80), the device comprising at least one double-opening region (42), in which at least one upper opening of the plurality of upper openings (70) or one lower opening of the plurality of lower openings (80) overlaps at least partially one middle opening (90) of the middle sheet (9), no opening being provided on one of the lower sheet and upper sheet in said at least one double-opening region,

the device further comprising an upper overcoat sheet (20) and a lower overcoat sheet (21) both extending in a horizontal plane (X, Y) and respectively arranged on top of the upper sheet (7) and below the lower sheet (8).

2. The device according to claim 1, the device further comprising a plurality of upper electrodes (10a) on the upper sheet (7) connected by a network of upper conductive leads (11) of the upper sheet (7) in a matrix arrangement such that each upper conductive lead (11) connects a row of upper electrodes (10a) substantially aligned along a transverse direction (Y) of the horizontal plane (X, Y),

and wherein the upper openings (70) extend substantially along the transverse direction (Y).

3. The device according to anyone of claims 1 to 2, the device further comprising a plurality of lower electrodes (10b) on the lower sheet (8) connected by a network of lower conductive leads (12) of the lower sheet (8) in a matrix arrangement such that each lower conductive lead (12) connects a column of lower electrodes (10b) substantially aligned along a longitudinal direction (X) of the horizontal plane (X, Y), and wherein the lower openings (80) extend substantially along the transverse direction (Y).

4. The device according to anyone of claims 1 to 3, wherein at least one of the upper openings (70) extends along a majority of a transversal extend of the device (1).

5. The device according to anyone of claims 1 to 4, wherein at least one of the upper openings (70) is an opening with a closed contour (70a).

6. The device according to anyone of claims 1 to 5, further comprising at least one ground plane (18, 19) extending in the horizontal plane (X, Y) over a majority of the device (1).

7. The device according to claim 6, further comprising an upper ground plane (18) on the upper sheet (7) and a lower ground plane (19) on the lower sheet (8) arranged such that the upper electrodes (10a), the middle sheet (9) and the lower electrodes (10b) being arranged between the lower ground plane (19) and the upper ground plane (18).

8. The device according to anyone of claims 1 to 7, wherein the flexible middle sheet (9) is insulating and wherein the force sensors (10) are capacitive force sensors.

9. The device according to anyone of claims 1 to 8, wherein at least a portion of the upper overcoat sheet (20) is able to slide in the horizontal plane (X, Y) with regard to the upper sheet (7).

10. The device according to anyone of claims 1 to 9, wherein the flexible pressure mapping device (1) is an insole for insertion into an article of footwear or is integrated in an article of footwear.

11. The device according to claims 9 and 10, further comprising an upper contacting layer (24) on top of the upper overcoat sheet (20) of the device (1), said upper contacting layer (24) being elastic and adapted to be engaged by a foot of a wearer.

12. A system (1000) for monitoring pressure comprising:

- a flexible pressure mapping device (1) according to anyone of claims 1 to 11, and
- a remote server (100) able to communicate with the control and transmit electronics (14) of the device (1) to receive pressure related data from the device (1).

**Patentansprüche**

1. Flexible Druckabbildungsvorrichtung (1), umfassend:

- eine flexible obere Schicht (7) und eine flexible untere Schicht (8), die einander zugewandt sind;
- eine flexible mittlere Schicht (9), die sich zwischen der oberen Schicht (7) und der unteren Schicht (8) erstreckt;
- eine Vielzahl von Kraftsensoren (10), die mindestens eine obere Elektrode (10a) auf der oberen Schicht (7) und mindestens eine untere Elektrode (10b) auf der unteren Schicht (8) umfassen, wobei die mindestens eine obere Elektrode (10a) der mindestens einen unteren Elektrode (10b) zugewandt ist, und von der unteren Elektrode (10b) mindestens durch die mittlere Schicht (9) getrennt ist;
- mindestens eine obere leitende Leitung (11) auf der oberen Schicht (7), die elektrisch mit der mindestens einen oberen Elektrode (10a) verbunden ist, und mindestens eine untere leitende Leitung (12) auf der unteren Schicht (8), die elektrisch mit der mindestens einen unteren Elektrode (10b) verbunden ist;
- eine Steuerungs- und Übertragungselektronik (14), die elektrisch mit der mindestens einen oberen leitenden Leitung (11) und mit der mindestens einen unteren leitenden Leitung (12) verbunden ist,
wobei die obere Schicht (7) eine Vielzahl von oberen Öffnungen (70) umfasst, und die untere Schicht (8) mindestens teilweise mindestens eine der oberen Öffnungen (70) überlappt,
wobei die untere Schicht (8) eine Vielzahl von unteren Öffnungen (80) umfasst, wobei die Vorrichtung mindestens eine Doppelöffnungsregion (42) umfasst, in der mindestens eine obere Öffnung der Vielzahl von oberen Öffnungen (70) oder eine untere Öffnung der Vielzahl von unteren Öffnungen (80) mindestens teilweise eine mittlere Öffnung (90) der mittleren Schicht (9) überlappt, wobei keine Öffnung an einer von der unteren Schicht und oberen Schicht in der mindestens einen Doppelöffnungsregion bereitgestellt ist,
wobei die Vorrichtung weiter eine obere Überzugsschicht (20) und eine unter Überzugsschicht (21) umfasst, die sich beide in einer horizontalen Ebene (X, Y) erstrecken und jeweils an der Oberseite der oberen Schicht (7) und unter der unteren Schicht (8) angeordnet sind.

2. Vorrichtung nach Anspruch 1, wobei die Vorrichtung weiter eine Vielzahl von oberen Elektroden (10a) auf der oberen Schicht (7) umfasst, die durch ein Netzwerk von oberen leitenden Leitungen (11) der oberen Schicht (7) in einer Matrixanordnung verbunden sind, sodass jede obere leitende Leitung (11) eine Reihe von oberen Elektroden (10a) verbindet, die im Wesentlichen entlang einer Querrichtung (Y) der horizontalen Ebene (X, Y) angeordnet sind,
und wobei sich die oberen Öffnungen (70) im Wesentlichen entlang der Querrichtung (Y) erstrecken.

3. Vorrichtung nach einem der Ansprüche 1 bis 2, wobei die Vorrichtung weiter eine Vielzahl von unteren Elektroden (10b) auf der unteren Schicht (8) umfasst, die durch ein Netzwerk von unteren leitenden Leitungen (12) der unteren Schicht (8) in einer Matrixanordnung verbunden sind, sodass jede untere leitende Leitung (12) eine Spalte von unteren Elektroden (10b) verbindet, die im Wesentlichen entlang einer Längsrichtung (X) der horizontalen Ebene (X, Y) angeordnet sind,
und wobei sich die unteren Öffnungen (80) im Wesentlichen entlang der Querrichtung (Y) erstrecken.

4. Vorrichtung nach einem der Ansprüche 1 bis 3, wobei sich mindestens eine der oberen Öffnungen (70) entlang eines Großteils einer Quererstreckung der Vorrichtung (1) erstreckt.

5. Vorrichtung nach einem der Ansprüche 1 bis 4, wobei mindestens eine der oberen Öffnungen (70) eine Öffnung mit einer geschlossenen Kontur (70a) ist.

6. Vorrichtung nach einem der Ansprüche 1 bis 5, weiter mindestens eine Grundebene (18, 19) umfassend, die sich in der horizontalen Ebene (X, Y) über einen Großteil der Vorrichtung (1) erstreckt.

7. Vorrichtung nach Anspruch 6, weiter umfassend eine obere Grundebene (18) auf der oberen Schicht (7) und eine untere Grundebene (19) auf der unteren Schicht (8), derart angeordnet, dass die oberen Elektroden (10a), die mittlere Schicht (9) und die unteren Elektroden (10b) zwischen der unteren Grundebene (19) und der oberen Grundebene (18) angeordnet sind.

8. Vorrichtung nach einem der Ansprüche 1 bis 7, wobei die flexible mittlere Schicht (9) isolierend ist und wobei die Kraftsensoren (10) kapazitive Kraftsensoren sind.

9. Vorrichtung nach einem der Ansprüche 1 bis 8, wobei mindestens ein Abschnitt der oberen Überzugsschicht (20) imstande ist, in der horizontalen Ebene (X, Y) in Bezug auf die obere Schicht (7) zu gleiten.

10. Vorrichtung nach einem der Ansprüche 1 bis 9, wobei die flexible Druckabbildungsvorrichtung (1) eine Innensohle zum Einführen in einen Fußbekleidungsartikel ist oder in einem Fußbekleidungsartikel integriert ist.

11. Vorrichtung nach den Ansprüchen 9 und 10, weiter eine obere Kontaktlage (24) an der Oberseite der oberen Überzugsschicht (20) der Vorrichtung (1) umfassend, wobei die obere Kontaktlage (24) elastisch und angepasst ist, um von einem Fuß eines Trägers belegt zu werden.

12. System (1000) zum Überwachen von Druck, umfassend:

- eine flexible Druckabbildungsvorrichtung (1) nach einem der Ansprüche 1 bis 11, und
- einen entfernten Server (100), der imstande ist, mit der Steuerungs- und Übertragungselektronik (14) der Vorrichtung (1) zu kommunizieren, um druckbezogene Daten von der Vorrichtung (1) zu empfangen.

**Revendications**

1. Dispositif flexible de cartographie de pression (1) comprenant :

- une feuille supérieure flexible (7) et une feuille inférieure flexible (8) se faisant face;
- une feuille médiane flexible (9) s'étendant entre la feuille supérieure (7) et la feuille inférieure (8);
- une pluralité de capteurs de force (10) comprenant au moins une électrode supérieure (10a) sur la feuille supérieure (7) et au moins une électrode inférieure (10b) sur la feuille inférieure (8), ladite au moins une électrode supérieure (10a) faisant face à ladite au moins une électrode inférieure (10b) et étant séparé de ladite électrode inférieure (10b) au moins par la feuille médiane (9);
- au moins un fil conducteur supérieur (11) sur la feuille supérieure (7), connecté électriquement à l'au moins une électrode supérieure (10a), et au moins un fil conducteur inférieur (12) sur la feuille inférieure (8), électriquement connecté à l'au moins une électrode inférieure (10b);
- un électronique de commande et de transmission (14) connecté électriquement à l'au moins un fil conducteur supérieur (11) et à l'au moins un fil conducteur inférieur (12),

dans lequel la feuille supérieure (7) comprend une pluralité d'ouvertures supérieures (70), et la feuille inférieure (8) chevauche au moins partiellement au moins une desdites ouvertures supérieures (70), la feuille inférieure (8) comprenant une pluralité d'ouvertures inférieures (80), le dispositif comprenant au moins une région à double ouverture (42), dans laquelle au moins une ouverture supérieure de la pluralité d'ouvertures supérieures (70) ou une ouverture inférieure de la pluralité d'ouvertures inférieures (80) chevauche au moins partiellement une ouverture médiane (90) de la feuille médiane (9), aucune ouverture n'étant prévue sur l'une des feuilles inférieure et supérieure dans ladite au moins une région à double ouverture, le dispositif comprenant en outre une feuille de revêtement supérieure (20) et une feuille de revêtement inférieure (21) s'étendant toutes les deux dans un plan horizontal (X, Y) et disposées respectivement sur le dessus de la feuille supérieure (7) et sous la feuille inférieure (8).

2. Le dispositif selon la revendication **1,** le dispositif comprenant en outre une pluralité d'électrodes supérieures (10a) sur la feuille supérieure (7) connectées par un réseau de fils conducteurs supérieurs (11) de la feuille supérieure (7) dans un agencement matricielle telle que chaque fil conducteur supérieur (11) relie une rangée d'électrodes supérieures (10a) sensiblement alignées le long d'une direction transversale (Y) du plan horizontal (X, Y), et dans lequel les ouvertures supérieures (70) s'étendent sensiblement le long de la direction transversale (Y).

3. Le dispositif selon l'une quelconque des revendications **1** à **2,** le dispositif comprenant en outre une pluralité d'électrodes inférieures (10b) sur la feuille inférieure (8) connectées par un réseau de fil conducteurs inférieurs (12) de la feuille inférieure (8) dans un agencement matriciel tel que chaque fil conducteur inférieur (12) relie une colonne d'électrodes inférieures (10b) sensiblement alignées selon une direction longitudinale (X) du plan horizontal (X, Y), et dans lequel les ouvertures inférieures (80) s'étendent sensiblement le long de la direction transversale (Y).

4. Dispositif selon l'une quelconque des revendications **1** à **3,** dans lequel au moins une des ouvertures supérieures (70) s'étend le long d'une majorité d'une extension transversale du dispositif (1).

5. Dispositif selon l'une quelconque des revendications **1** à **4,** dans lequel au moins une des ouvertures supérieures (70) est une ouverture avec un contour fermé (70a).

6. Dispositif selon l'une quelconque des revendications **1** à **5,** comprenant en outre au moins un plan de masse (18, 19) s'étendant dans le plan horizontal (X, Y) sur une majorité du dispositif (1).

7. Dispositif selon la revendication **6,** comprenant en outre un plan de masse supérieur (18) sur la feuille supérieure (7) et un plan de masse inférieur (19) sur la feuille inférieure (8) agencés de telle sorte que les électrodes supérieures (10a), la feuille médiane (9) et les électrodes inférieures (10b) soient disposées entre le plan de masse inférieur (19) et le plan de masse supérieur (18).

8. Dispositif selon l'une quelconque des revendications **1** à **7,** dans lequel la feuille médiane flexible (9) est isolante et dans lequel les capteurs de force (10) sont des capteurs de force capacitifs.

9. Dispositif selon l'une quelconque des revendications **1** à **8,** dans lequel au moins une partie de la feuille de revêtement supérieure (20) est capable de coulisser dans le plan horizontal (X, Y) par rapport à la feuille supérieure (7).

10. Dispositif selon l'une quelconque des revendications **1** à **9,** dans lequel le dispositif flexible de cartographie de pression (1) est une semelle intérieure à insérer dans un article chaussant ou est intégré dans un article chaussant.

11. Dispositif selon les revendications **9** et **10,** comprenant en outre une couche de contact supérieure (24) sur le dessus de la feuille de revêtement supérieure (20) du dispositif (1), ladite couche de contact supérieure (24) étant élastique et adaptée pour être engagée par un pied d'un porteur.

12. Système (1000) pour surveiller la pression comprenant :

- un dispositif flexible de cartographie de pression (1) selon l'une quelconque des revendications **1** à **11,** et
- un serveur distant (100) apte à communiquer avec l'électronique de commande et de transmission (14) du dispositif (1) pour recevoir des données liées à la pression du dispositif (1).

FIG. 1

FIG. 2

**FIG. 3**

FIG. 4A                    FIG. 4B

EP 3 235 428 B1

9a

90

90

9b

9

FIG. 4C

200

FIG. 4D

20

18

22, 23

22a, 23a

22a, 23a

**FIG. 4E**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009070782 A
- US 2013213145 A

- WO 2009089406 A